# EUROPÄISCHE PATENTSCHRIFT

(11) **EP 3 656 338 B1**
(45) Veröffentlichungstag und Bekanntmachung des Hinweises auf die Patenterteilung: **18.09.2024**
(21) Anmeldenummer: 19208881.3
(22) Anmeldetag: 13.11.2019
(51) Int. Cl.: A61C 1/00, A61L 9/20

(54) **DESINFEKTIONSVORRICHTUNG SOWIE KOMPRESSORSYSTEM, VERBINDUNGSEINRICHTUNG UND BEHANDLUNGSEINRICHTUNG MIT EINER SOLCHEN**
DISINFECTION DEVICE AND COMPRESSOR SYSTEM, CONNECTION DEVICE AND TREATMENT DEVICE COMPRISING SAME
DISPOSITIF DE DÉSINFECTION AINSI QUE SYSTÈME DE COMPRESSEUR, AGENCEMENT DE RACCORDEMENT ET AGENCEMENT DE TRAITEMENT DOTÉ D'UN TEL DISPOSITIF

(30) Priorität: 26.11.2018 DE 102018129811
(43) Veröffentlichungstag der Anmeldung: 27.05.2020
(73) Patentinhaber: Dürr Dental SE, 74321 Bietigheim-Bissingen (DE)
(72) Erfinder: Dürrstein, Martin, 74321 Bietigheim-Bissingen (DE); Hägele, Andreas, 71384 Weinstadt (DE); Bauer, Walter, 71735 Eberdingen-Nussdorf (DE); Schmidtke, Jürgen, 75203 Königsbach-Stein (DE)
(74) Vertreter: Frenkel, Matthias Alexander

(56) Entgegenhaltungen:
- WO-A1-2005/011753
- US-A1- 2003 190 254
- US-A1- 2015 165 078
- US-A1- 2016 311 699

## Beschreibung

Die Erfindung betrifft eine Desinfektionsvorrichtung mit mindestens einer Abstrahlvorrichtung.

Die Erfindung betrifft auch ein Kompressorsystem zum Bereitstellen von Druckluft für dentalmedizinische Anwendungen, eine dentale Behandlungseinrichtung mit einer Drucklufteinrichtung, sowie eine Verbindungseinrichtung mit einem oder mehreren Rohrabschnitten zum Führen von Druckluft für dentalmedizinische Anwendungen mit einer solchen Desinfektionseinrichtung.

Aus der DE 20 2016 104 823 U1 ist eine Entkeimungsvorrichtung zum Entkeimen von Transport- und Bearbeitungsflächen mit einer Leuchtquelle bekannt, wobei die Leuchtquelle einen Röhrenkörper aufweist, der eine emittierende UV-Quelle mit elektrischen Anschlüssen umfasst. Die hier beschriebene Entkeimungsvorrichtung arbeitet in einer Umgebung mit Raumluft. Darüber hinaus ist die hier beschriebene Entkeimungsvorrichtung mit der entsprechenden Leuchtquelle nur für bestimmte Anwendungsgebiete wie die Textilreinigung konzipiert.

Die DE 20 2014 012 690 offenbart eine Wasseraufbereitungsvorrichtung für eine dentale Behandlungseinheit, wobei das Wasser durch Ultrafiltration an einem Ultrafiltrationsfilter entkeimt wird.

In der Dentalmedizin werden besonders hohe Hygieneanforderungen an Produkte, Betriebsstoffe und Materialien gestellt. Dentale Druckluft wird schließlich unter anderem auch bei der Behandlung am Patienten verwendet, beispielsweise zum Ausblasen von Kavitäten oder zur Zahnreinigung, z.B. bei der Verwendung von Pulverstahlgeräten. Je nach Anwendungsfall kann die Druckluft dabei mit Körperöffnungen wie Zahntaschen oder Wurzelkanälen direkt in Berührung kommen. Daher ist ein besonders hoher Hygienegrad gerade bei dentaler Druckluft besonders erstrebenswert.

Um die Hygieneanforderungen für dentale Druckluft umzusetzen ist die Verwendung von Bakterienfiltern in der Ansaugluft, von Trockenluftanlagen und von Partikelfilter bekannt. Bei Kompressoren für dentale Druckluft ist durch den hohen Kompressionsdruck und durch die hohe Temperatur bei der Verdichtung davon auszugehen, dass die erzeugte Druckluft frei von Bakterien und Viren ist. Die erzeugte Druckluft kann z.B. über eine Verschlauchung, über ein oder mehrere Filter, über einen Kühler und eine Trockenluftanlage in einen Drucklufttank geführt und dort gespeichert werden. Je nach Wartungszustand, Funktion, Stillstandszeiten und Feuchtigkeitsgehalt kann nicht ausgeschlossen werden, dass es beispielsweise im Drucklufttank zu einer Verkeimung der gespeicherten Luft kommt. Unter Umständen kann auch eine retrograde Luftverkeimung auftreten, die von der dentalen Behandlungseinrichtung herkommend ausgelöst werden kann, beispielsweise ausgehend von Leitungen bzw. Rohren in oder am Behandlungsstuhl.

Aus der US 2015/0165078 A1 ist daher ein Druckluftkompressor im dentalen Bereich mit einem Drucklufttank bekannt, in dessen Inneren Hochleistungs-UV-LEDs angeordnet sind, mit deren Hilfe die Innenluft mit keimtötendem UV Licht geflutet wird. Um eine ausreichende biologische Wirkung zu erreichen, sind jedoch starke UV-LEDs notwendig, die in jenen Bereich gerichtet werden, in welchem sich Flüssigkeit, insbesondere Wasser ansammelt.

Aus der US 2003/0190254 A1 ist eine UV-Desinfektionsvorrichtung für ein Kompressorsystem bekannt, bei der am Auslass eines Drucklufttanks eine UV-Kammer angeordnet ist. In die UV-Kammer ragt eine UV-Lampe in einer Quarzglasröhre, die einem hohen Druck Stand hält und aus welcher das UV-Licht radial austritt.

Aus der WO 2005/011753 A1 ist eine In-Line-Desinfektionsvorrichtung bekannt, bei welcher ein Gasstrom in einer Quarzglasröhre geleitet wird. Zur Desinfektion des Gasstroms wird UV-Licht parallel zur Ausbreitungsrichtung der Quarzglasröhre eingekoppelt, damit das UV-Licht möglichst lange mit dem Gasstrom interagiert.

Aus der US 2016/0311699 A1 ist ein portabler Koffer zur Dentalbehandlung in entlegenen Gebieten bekannt. Dabei wird wahlweise ein Wassertank im Koffer oder der Innenraum des Koffers mit UV-Licht desinfiziert. Der Koffer ist mit einer reflektierenden Innenauskleidung versehen, um die keimtötende Wirkung zu maximieren.

Aufgabe der vorliegenden Erfindung ist es, zumindest eine Desinfektionsvorrichtung bereitzustellen, die gegenüber dem Stand der Technik weiterentwickelt und insbesondere zur Entkeimung von dentaler Druckluft geeignet ist.

Diese Aufgabe wird gelöst durch eine Desinfektionsvorrichtung gemäß Anspruch 1. Die Desinfektionsvorrichtung weist ein rohrförmiges Druckgefäß mit einem Innenraum zur Aufnahme eines Mediums auf, wobei die mindestens eine Abstrahlvorrichtung der Desinfektionsvorrichtung derart angeordnet ist, dass sie Strahlung in den Innenraum des Druckgefäßes abgibt, und wobei ein oder mehrere Oberflächen des Innenraums des Druckgefäßes zumindest teilweise als Reflexionsfläche ausgebildet sind, vorzugsweise mit einem Reflexionsgrad von mehr als 90%. Erfindungsgemäß wird eine besonders zuverlässige Entkeimung direkt im Druckgefäß ermöglicht. Etwaige im Druckgefäß befindliche Bakterien oder Viren können abgetötet werden, wodurch beispielsweise auch einer retrograden Verkeimung sicher entgegengewirkt werden kann. Die Erfindung ermöglicht es, ein Medium bei einem definierten Volumenstrom und einer entsprechenden Fließgeschwindigkeit besonders zuverlässig zu entkeimen.

Dabei weist die mindestens eine Abstrahlvorrichtung eine UVC-LED-Strahlungsquelle auf. Derart wird eine besonders zuverlässige und zugleich umweltfreundliche Desinfektion ermöglicht. Eine derartige Desinfektionsvorrichtung kann auch ohne den Einsatz von Chemikalien wirken.

Eine LED als Strahlungsquelle zeichnet sich durch eine hohe Betriebssicherheit aus und ist für den Einsatz im medizinischen bzw. zahnmedizinischen Bereich besonders geeignet.

Es kann zweckmäßig sein, dass die Strahlungsquelle der Abstrahlvorrichtung in einem Hohlspiegel angeordnet ist. Derart kann die Abstrahlcharakteristik verbessert werden.

Die Abstrahlvorrichtung ist derart ausgebildet, dass eine Hauptrichtungskomponente der abgegebenen Strahlung parallel zu einer Längsachse des Druckgefäßes ausgerichtet ist. Derart kann die Desinfektionswirkung der Strahlung gesteigert werden.

Die mindestens eine Abstrahlvorrichtung ist im Bereich einer ersten Seite des Innenraums angeordnet, wobei eine zweite, dieser ersten Seite entlang der Längsachse betrachtet gegenüberliegende Seite des Innenraums eine Reflexionsfläche für die Strahlung aufweist. Derart kann weitestgehend vermieden werden, dass von der mindestens einen Strahlungsquelle abgegebene Strahlung an der Seite des Druckgefäßes, die der Strahlungsquelle gegenüberliegt, zu einem Großteil absorbiert wird.

Die Reflexionsfläche ist durch den Einbau einer Spiegelfolie und/oder eines Spiegelblechs oder durch Bedampfen hergestellt.

In weiter vorteilhafter Ausgestaltung weisen mindestens zwei sich gegenüberliegende Seiten des Innenraums Reflexionsflächen für die Strahlung auf.

Zur geeigneten Ausrichtung der Strahlung im Druckgefäß ist es günstig, wenn mindestens eine der Reflexionsflächen in Form eines Hohlspiegels ausgebildet ist.

Mit Vorteil ist mindestens eine Reflexionsfläche durch eine auf ein Trägermaterial aufgebrachte Reflexionsschicht ausgebildet.

Reflexionsverluste können gering gehalten werden, wenn die Reflexionsschicht zumindest teilweise aus Aluminium besteht.

In einer vorteilhaften Ausgestaltung ist das Druckgefäß als Tank mit mindestens einem Durchlass zum Ein- und Ausströmen des Mediums ausgebildet.

In einer weiteren vorteilhaften Ausgestaltung ist das Druckgefäß als rohrförmiger Druckbehälter mit zwei oder mehr Durchlässen für das Medium ausgebildet.

Vorzugsweise ist das Druckgefäß als Druckluftgefäß für dentale Druckluft ausgebildet.

Die der Erfindung zugrundeliegende Aufgabe wird auch gelöst durch ein Kompressorsystem zum Bereitstellen von Druckluft für dentalmedizinische Anwendungen mit einer Desinfektionsvorrichtung, die gemäß der Erfindung oder einer ihrer Ausgestaltungen ausgebildet ist. Wesentliche Vorteile ergeben sich dabei in Analogie zu den Vorteilen der Desinfektionsvorrichtung.

Die der Erfindung zugrundeliegende Aufgabe wird auch gelöst durch eine dentale Behandlungseinrichtung mit einer Drucklufteinrichtung, die eine Desinfektionsvorrichtung aufweist, die gemäß der Erfindung oder einer ihrer Ausgestaltungen ausgebildet ist. Wesentliche Vorteile ergeben sich dabei in Analogie zu den Vorteilen der Desinfektionsvorrichtung.

Die der Erfindung zugrundeliegende Aufgabe wird auch gelöst durch eine Verbindungseinrichtung mit einem oder mehreren Rohrabschnitten zum Führen von Druckluft für dentalmedizinische Anwendungen, wobei mindestens ein Rohrabschnitt eine Desinfektionsvorrichtung aufweist, die gemäß der Erfindung oder einer ihrer Ausgestaltungen ausgebildet ist. Wesentliche Vorteile ergeben sich dabei in Analogie zu den Vorteilen der Desinfektionsvorrichtung.

Weitere vorteilhafte Ausgestaltungen der Erfindung ergeben sich aus der nachfolgenden Beschreibung. Dabei werden Ausführungsbeispiele der Erfindung, ohne hierauf beschränkt zu sein, anhand der Zeichnung näher erläutert. Es zeigen in vereinfachter, schematischer Darstellung:
- Figur 1: ein Ausführungsbeispiel einer erfindungsgemäßen Desinfektionsvorrichtung in Schnittansicht;
- Figur 2: ein weiteres Ausführungsbeispiel einer erfindungsgemäßen Desinfektionsvorrichtung in Schnittansicht;
- Figur 3: ein weiteres Ausführungsbeispiel einer erfindungsgemäßen Desinfektionsvorrichtung in Schnittansicht;
- Figur 4: eine Abstrahlvorrichtung in Schnittansicht;
- Figur 5: eine weitere Abstrahlvorrichtung in Schnittansicht;
- Figur 6: eine weitere Abstrahlvorrichtung in Schnittansicht;
- Figur 7: ein Druckluftsystem in stark vereinfachter schematischer Ansicht.

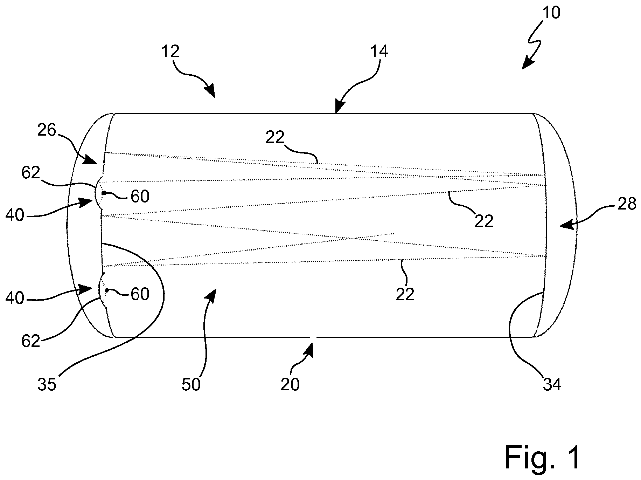

Die Figuren 1 bis 3 zeigen Beispiele für eine Desinfektionsvorrichtung 10 zur Desinfektion eines Mediums, wie beispielsweise Luft oder Flüssigkeit, im Innenraum 50 eines Druckgefäßes 12. Besonders geeignet ist die Desinfektionsvorrichtung 10 für Druckluft, die in der Medizin oder Dentalmedizin verwendet wird. Die Figuren 1 bis 3 zeigen beispielhaft Druckgefäße 12, die vorzugsweise als Druckluftgefäße für dentale Druckluft ausgebildet sind.

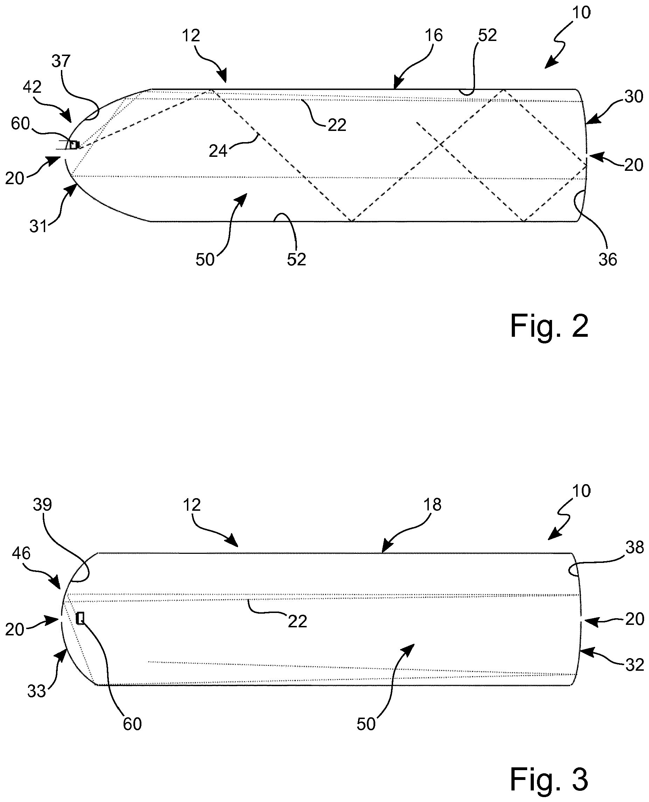

Dabei zeigt Figur 1 ein Druckgefäß 12, welches vorteilhaft als Druckspeicher an einem Kompressor geeignet ist. Die Figuren 2 und 3 zeigen hingegen Druckgefäße 12, welche vorteilhaft für den Einbau in einem Behandlungsstuhl oder in Verbindungseinrichtungen geeignet sind.

Die in den Figuren 1 bis 3 beispielhaft dargestellten Desinfektionsvorrichtungen 10 sind mit Abstrahlvorrichtungen 40, 42 bzw. 46 derart ausgestattet, dass durch die Abstrahlvorrichtungen 40, 42 bzw. 46 eine gerichtete Strahlung in den Innenraum 50 des jeweiligen Druckgefäßes 12 injiziert wird. Die Druckgefäße 12 weisen in ihrem Innenraum 50 Reflexionsflächen 34, 35, 36, 37, 38, 39, 52 zur Reflexion der injizierten Strahlung auf. Beispielhaft sind einige Strahlenwege 22 der von den Abstrahlvorrichtungen 40, 42, 44, 46 abgegebenen Strahlung in den Figuren 1 bis 3 angedeutet. Die Strahlenwege 22 sind der besseren Übersicht halber in den Zeichnungen nicht durchgehend mit Bezugszeichen versehen.

Figur 1 zeigt eine Desinfektionsvorrichtung 10 mit einem als Tank 14 ausgebildeten Druckgefäß 12. Im gezeigten Beispiel ist das Druckgefäß 12 ein Drucklufttank mit einem Durchlass 20 zum Zu- und Abführen von Luft. Der Tank 14 ist im Wesentlichen zylinderförmig ausgebildet und weist an seinen beiden Endseiten jeweils ein Bodenteil 26, 28 auf.

Die Bodenteile 26, 28 können beispielsweise als sogenannte Klöpperböden ausgebildet sein. Als Drucklufttank für die Dentalmedizin kann der Tank 14 beispielsweise ein Volumen von ca. 60 Liter bis ca. 90 Liter aufweisen. Der im Beispiel gezeigte Tank 14 weist eine Länge auf, die deutlich größer als sein Durchmesser ist. Im Beispiel misst die Länge des Tanks 14 ungefähr das Doppelte des Durchmessers.

In dem in Figur 1 gezeigten Beispiel sind im Bereich des ersten Bodenteils 26 mehrere Abstrahlvorrichtungen 40 vorgesehen. Die gezeigten Abstrahlvorrichtungen 40 weisen jeweils eine Strahlungsquelle 60 auf, die in das Druckgefäß 12 abstrahlt. Als Strahlungsquellen 60 können UVC-Strahlquellen, vorzugsweise UVC-LEDs, verwendet werden.

Um innerhalb des Druckgefäßes 12 eine möglichst große Desinfektionswirkung zu erzielen (d.h. insbesondere eine große Luftmasse zu bestrahlen), wird die Strahlung zumindest im Wesentlichen in Längsrichtung zwischen einander gegenüberliegend angeordneten Reflexionsflächen 34, 35 vorzugsweise mehrfach hin und her reflektiert. Diese Reflexionsflächen 34, 35 sind an den Innenseiten der Bodenteile 28 bzw. 26 angeordnet. Entsprechende Strahlenwege 22 sind in der Zeichnung angedeutet. Vorzugsweise bilden die Reflexionsflächen 34, 35 jeweils einen Hohlspiegel aus. Durch die vorangehend beschriebene Reflexion der abgegebenen Strahlung wird eine deutliche Wirkungssteigerung erreicht. Die Wirkungssteigerung ist dann besonders groß, wenn die Strahlung zwischen ihren Reflexionspunkten eine möglichst lange Strecke zurücklegt und sich so die Trefferwahrscheinlichkeit der Photonen mit den Mikroorganismen vervielfacht.

Wie in der Zeichnung angedeutet, können die Abstrahlvorrichtungen 40 jeweils einen Reflektor 62 für die Strahlungsquelle 60 aufweisen. Der Reflektor 62 kann vorzugsweise als Hohlspiegel, z.B. als Parabolspiegel, ausgebildet sein. Im gezeigten Beispiel sind die Strahlungsquellen 60 jeweils in einem inneren Reflektor 62, d.h. dem Reflektor 62 der Strahlungsquelle 60 angeordnet. Dieser innere Reflektor 62 ist seinerseits in einem äußeren Reflektor angeordnet, der durch die Reflexionsfläche 35 an der Innenseite des Bodenteils 26 des Druckgefäßes 12 ausgebildet wird.

Das Druckgefäß 12 kann zumindest eine in der Zeichnung nicht näher dargestellte Revisionsöffnung aufweisen, um bei Bedarf den Zugang zu der mindestens einen Strahlungsquelle 60 zu ermöglichen. Eine derartige Revisionsöffnung kann im Bodenteil 26 hinter der mindestens einen Strahlungsquelle 60 angeordnet sein. Ein elektrischer Anschluss der mindestens einen Strahlungsquelle 60 erfolgt vorzugsweise über das Bodenteil 26 hinter der Strahlungsquelle 60. Die Revisionsöffnung kann auch so ausgelegt sein, dass die Strahlungsquelle 60 mit ihren Zuleitungen direkt in der Revisionsöffnung ein- und ausbaubar fixiert ist.

Bei dem in Figur 1 gezeigten Ausführungsbeispiel bilden die Hohlspiegel förmigen Reflexionsflächen 34, 35 im Innenraum 50 des Druckgefäßes 12 eine Art Käfig für die Strahlung aus. Die Strahlung klingt dabei im Wesentlichen ab durch Verluste an den Reflexionsflächen 34, 35 bzw. durch Absorption in der Luft und an den Mikroorganismen. Um eine entsprechende Führung der Strahlung zu gewährleisten, sind die Hohlspiegel in hinreichender Weise sphärisch ausgeführt. Alternativ ist auch eine leicht asphärische Formung möglich.

Die Apertur der Hohlspiegel entspricht vorzugsweise ungefähr dem Durchmesser des Druckgefäßes 12, wobei auch eine um bis zu ca. 10 % kleinere Apertur nur wenig ungünstiger ist. Der Spiegelradius der Hohlspiegel beträgt mit Vorteil mindestens das ca. 1,5-fache der Behälterlänge, vorzugsweise das 2- bis 6-fache der Behälterlänge. Grundsätzlich ist es auch möglich, dass die Reflexionsflächen der Bodenteile 26, 28 eben ausgebildet sind. Dann ist jedoch höhere Strahlparallelität der Abstrahlvorrichtung notwendig, um eine vergleichbare Vervielfachung der Desinfektionswirkung zu erzielen.

Die Figuren 2 und 3 zeigen jeweils eine Desinfektionsvorrichtung 10 mit einem rohrförmigen Druckbehälter 16 bzw. 18 als Druckgefäß 12. Der Durchmesser des Druckbehälters 16 bzw. 18 ist dabei reduziert im Vergleich zu der in Figur 1 gezeigten Ausbildung als Tank 14. Dadurch ist dieser bevorzugt für den Einbau in Zuleitungen oder einem Behandlungsstuhl geeignet. Derartige rohrförmige Druckbehälter 16, 18 können im Wesentlichen zylinderförmig ausgebildet sein. Es ist auch möglich, dass die Druckbehälter 16, 18 eine im Wesentlichen einem Kegelstumpf entsprechende Geometrie aufweisen, d.h. dass ihr Mantel einen Winkel zwischen der Mantellinie und der Längsachse des Druckbehälters 16, 18 aufweist.

Die in den Figuren 2 und 3 gezeigten rohrförmigen Druckbehälter 16, 18 weisen jeweils zwei Endteile 30 und 31 bzw. 32 und 33 auf, die sich einander gegenüber liegen. In den beiden Endteilen 30, 31 bzw. 32, 33 ist jeweils ein Durchlass 20 angeordnet. Über die Durchlässe 20 kann dem Druckbehälter 16 bzw. 18 ein Medium zu- und/oder abgeführt werden. In den gezeigten Beispielen sind die Durchlässe 20 zentral in den Endteilen 30, 31 bzw. 32, 33 angeordnet.

Die Innenseiten der Endteile 30, 31, 32, 33 sind mit Reflexionsflächen 36, 37 bzw. 38, 39 ausgestattet. Im Bereich eines ersten Endteils 31, 33 ist jeweils mindestens eine Abstrahlvorrichtungen 42 bzw. 46 vorgesehen. Das erste Endteil 31, 33 der Druckbehälter 16 bzw. 18 weist eine Krümmung auf. Die an der Innenseite des ersten Endteils 31, 33 angeordnete Reflexionsfläche 37 bzw. 39 bildet in den gezeigten Beispielen einen Hohlspiegel aus. Der Hohlspiegel kann dabei vorzugsweise in Form eines Parabolspiegels ausgebildet sein. Das zweite Endteil 30, 32 weist eine Reflexionsfläche 36 bzw. 38 auf, die beispielsweise eine leicht gekrümmte oder auch eine ebene Spiegelfläche für die Strahlung ausbilden kann.

Insgesamt ist die Geometrie des Ausführungsbeispiels gemäß Figur 1 günstiger als die der Ausführungsbeispiele gemäß den Figuren 2 und 3. Der Multiplikationseffekt für die Strahlung ist bei dem in Figur 1 gezeigten Ausführungsbeispiel größer.

Gegegenüber dem in Figur 1 gezeigten Beispiel wird bei den in Figur 2 und 3 gezeigten Beispielen die Strahlung bei jedem "Durchlauf" zweimal an den Reflexionsflächen 37 bzw. 39 und einmal an der Reflexionsfläche 36 bzw. 38, also insgesamt dreimal anstatt zweimal reflektiert.

Bei dem in Figur 2 gezeigten Ausführungsbeispiel der Desinfektionsvorrichtung 10 wird die Strahlung wesentlich häufiger am Mantel des Druckgefäßes 12 reflektiert als bei dem Ausführungsbeispiel gemäß Figur 1. Die häufigere Reflexion der Strahlung schwächt die Strahlung ab, wodurch die Desinfektionswirkung verringert wird. Beispielhaft ist ein Strahlenweg 24 mit mehrfacher Reflexion am Mantel in Figur 2 mit gestrichelter Linie angedeutet. Um einer Abschwächung der Strahlung am Mantel des Druckgefäßes 12 entgegenzuwirken, ist die Innenseite des Mantels des beispielhaft in Figur 2 gezeigten rohrförmigen Druckbehälters 16 mit einer Reflexionsfläche 52 ausgestattet. Derart wird einer Absorption der Strahlung an der Innenseite des Mantels entgegengewirkt.

Die Reflexionsflächen 34, 35, 36, 37, 38, 39, 52 der in den Figuren 1 bis 3 gezeigten Druckgefäße 12 können durch eine auf ein Trägermaterial aufgebrachte Reflektorschicht bereitgestellt werden. Dabei kann ein Teil des Gehäuses des Druckgefäßes 12, also z.B. ein Bodenteil 26, 28 bzw. ein Endteil 30, 31, 32, 33 des Druckgefäßes 12, bei entsprechender Formgebung unmittelbar selbst als Trägermaterial oder als Auflage für das Trägermaterial verwendet werden.

Vorzugsweise besteht die Reflektorschicht aus Aluminium. Möglichst reines Aluminium ist als Reflexionsmaterial für UVC-Strahlung besonders gut geeignet, insbesondere für Strahlung mit einem Wellenlängenbereich zwischen ca. 250 nm und ca. 290 nm. Um die Haltbarkeit zu verbessern kann gegebenenfalls eine zusätzliche Schutzschicht vorgesehen werden.

Um die Herstellungskosten zu optimieren kann es beispielsweise bei Reflexionsflächen 52, die wie in Figur 2 veranschaulicht an der Innenseite des Mantels bzw. des Mittelteils eines rohrförmigen Druckbehälters 16 angeordnet sind, zweckmäßig sein, zum Herstellen der Reflexionsflächen 52 polierten Stahl, Edelstahl und/oder Aluminium jeweils mit bzw. ohne Schutzschicht zu verwenden.

Die Reflexionsflächen 34, 35, 36, 37, 38, 39, 52 können beispielsweise durch den Einbau von Spiegelfolien und/oder Spiegelblechen hergestellt werden. Die Reflexionsflächen 34, 35, 36, 37, 38, 39, 52 können beispielsweise auch durch Bedampfen von Oberflächen im Inneren des Druckgefäßes 12 hergestellt werden.

Ein in Form eines Tanks 14 (siehe Figur 1) ausgebildetes Druckgefäß 12, insbesondere ein Drucklufttank, kann beispielsweise hergestellt werden mit einem gerollten und verschweißten Stahlblech als Mittelteil und mit tiefgezogenen, sogenannten Klöpperböden als Endteilen 31, 33 an den Außenseiten, wobei die Klöpperböden mit dem Mittelteil verschweißt werden. Der Mittelteil bildet dabei den Mantel des im Wesentlichen Zylinder förmig ausgebildeten Tanks 14. Es erfolgt vorzugsweise eine Innenlackierung des Drucklufttanks zum Korrosionsschutz, beispielsweise mittels einer Lanze.

Die Reflexionsflächen 34, 35 können beispielsweise derart hergestellt werden, dass die geformte mindestens eine Reflexionsschicht mit den Klöpperboden z.B. mittels Punktschweißen verbunden wird. Gegebenenfalls ist die mindestens eine Reflexionsschicht an der relevanten Position mit einer Schutzfolie versehen. Diese Schutzfolie kann beispielsweise nach dem Verschweißen der beiden Klöpperböden mit dem Tankinnenteil und ggf. nach einer anschließenden Innenlackierung durch eine Revisionsöffnung entfernt werden. Alternativ kann die mindestens eine Reflexionsschicht erst nach dem Schweißen des Druckgefäß 12 aufgedampft werden. Das Druckgefäß 12 kann hierzu ggf. evakuiert werden und die Beschichtungsquellen werden durch eine oder mehrere Revisionsöffnungen eingebracht.

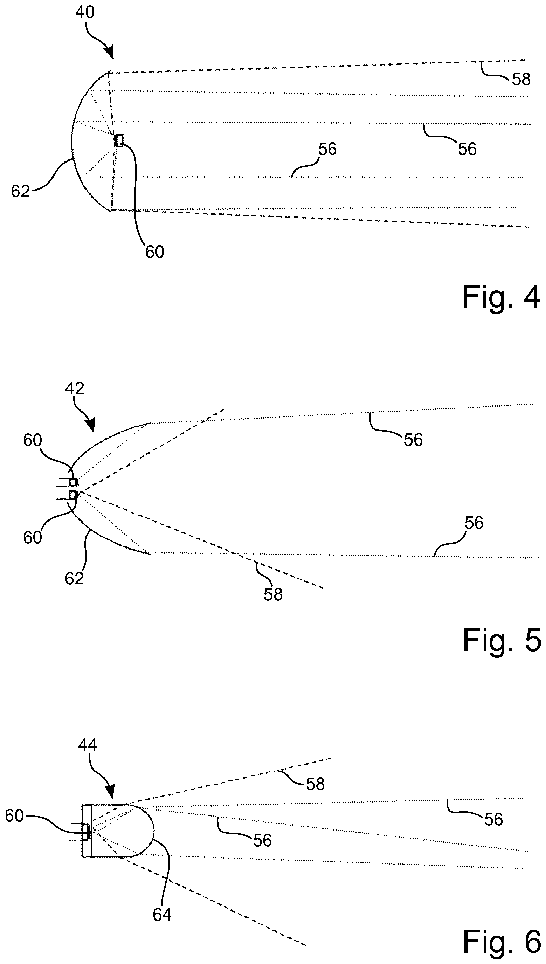

Die Figuren 4 bis 6 zeigen beispielhaft unterschiedlich ausgestaltete Abstrahlvorrichtungen 40, 42, 44, die ein oder mehrere Strahlungsquellen 60 aufweisen. Beispielhaft sind in den Figuren 4 bis 6 einige Strahlenwege 56, 58 der von den Strahlungsquellen 60 ausgehenden Strahlung angedeutet.

Die Figuren 4 und 5 zeigen Strahlungsquellen 60, die in einem als Hohlspiegel ausgeführten Reflektor 62 angeordnet sind, wobei die in den Figuren 4 und 5 gezeigten Abstrahlvorrichtungen 40, 42 als Injektorstrahler ausgebildet sein können. Die von den Strahlungsquellen 60 ausgehende Strahlung wird möglichst verlustfrei in den Reflektor 62 eingespeist, wobei durch Reflexion an dem als Hohlspiegel ausgebildeten Reflektor 62 ein gerichtetes Strahlenbündel geformt wird. Aufgabe derartiger Abstrahlvorrichtungen ist es, die von einer oder von mehreren dicht nebeneinander platzierten Strahlungsquellen 60 ausgehende Strahlung möglichst verlustfrei zu einem möglichst parallelen Strahlenbündel zu formen. Die Strahlrichtung des von der Abstrahlvorrichtung 40, 42 abgegebenen Strahlenbündels ist dabei vom Reflektor 62 weg gerichtet. Die primäre Strahlrichtung der Strahlenquellen 60 ist jedoch zwischen den beiden beispielhaften Abstrahlvorrichtungen 40 und 42 entgegengesetzt.

Bei der in Figur 4 gezeigten Abstrahlvorrichtung 40 wird ein sehr hoher Anteil der von der Strahlungsquelle 60 ausgehenden Strahlung direkt in den vorzugsweise als Parabolspiegel ausgebildeten Reflektor 62 gerichtet und dann von dort reflektiert.

Bei der in Figur 5 gezeigten Abstrahlvorrichtung 42 ist der ebenso vorzugsweise als Parabolspiegel ausgebildete Hohlspiegel des Reflektors 62 im Vergleich zu Figur 4 stärker gekrümmt. Im Gegensatz zu Figur 4 ist gemäß Figur 5 die Strahlrichtung der Strahlungsquellen 60 hauptsächlich direkt aus dem Reflektor 62 heraus gerichtet. In Figur 5 wird daher nicht alle von den Strahlungsquellen 60 abgegebene Strahlung von dem Reflektor 62 in die Strahlrichtung reflektiert.

Figur 6 zeigt eine Abstrahlvorrichtung 44 mit einer Strahlungsquelle 60 und mit einem Linsenkörper 64. Der Linsenkörper 64 ist strahlungsdurchlässig ausgebildet und bildet im gezeigten Beispiel ein Gehäuse für die Strahlungsquelle 60. Die von der Strahlungsquelle 60 abgegebene Strahlung wird am Linsenkörper 64 gebrochen. Um eine zweckmäßige und gute Strahlformung zu erreichen, muss der Linsenkörper 64 entsprechend groß und/oder die Emissionsfläche der Strahlungsquelle 60 entsprechend klein sein. Die in Figur 6 beispielhaft gezeigte Bauform ist vor allem im sichtbaren Wellenlängenbereich des Lichts besonders kostengünstig. Im UVC-Wellenlängenbereich trifft dies derzeit nur bedingt zu, wobei hier Fortschritte zu erwarten sind, welche eine derartige Bauform auch für den UVC-Wellenlängenbereich ermöglichen.

Der Emissionswinkel der in den Figuren 5 und 6 gezeigten Abstrahlvorrichtungen 42, 44 ist grösser als der Emissionswinkel der in Figur 4 gezeigten Abstrahlvorrichtung 40. In den Zeichnungen wird dieser Vergleich insbesondere durch die jeweils mit einer gestrichelten Linie gekennzeichneten beispielhaften Strahlenwege 58 verdeutlicht. Der höhere Emissionswinkel der in den Figuren 5 und 6 gezeigten Abstrahlvorrichtungen 42, 44 führt zu höheren Verlusten an den Innenwänden des Mantels bzw. des Mittelteils des Druckgefäßes 12. Um diesen Verlusten entgegenzuwirken, kann die Anzahl der in der Desinfektionsvorrichtung 10 verwendeten Abstrahlvorrichtungen 42, 44 erhöht werden. Alternativ oder zusätzlich kann, wie schon vorangehend im Zusammenhang mit Figur 2 beschrieben, die Innenwand des Mantels des Druckgefäßes 12 mit einer entsprechenden Reflexionsfläche 52 ausgestattet werden.

Die vorangehend an Hand der Figuren 2 und 3 beschriebenen Desinfektionsvorrichtungen 10 sind zwar bedingt durch höhere Verluste weniger leistungsfähig im Vergleich zu einer Desinfektionsvorrichtungen 10, wie sie beispielhaft in Figur 1 dargestellt ist. Eine geringere Leistungsfähigkeit ist meist auch noch trotz des Vorsehens mindestens einer geeigneten Reflexionsfläche 52 an der Innenwand des Mantels des Druckgefäßes 12 gegeben. Dennoch können die hier beschriebenen Desinfektionsvorrichtungen 10 mit einem rohrförmigen Druckbehälter 16, 18 für reduzierte Volumenströme ausreichen und mit vergleichsweise geringem Aufwand und kleinerem Durchmesser hergestellt werden. Reduzierte Volumenströme können beispielsweise an einzelnen Behandlungseinrichtungen 80 oder auch in einer Verbindungsleitung 76 auftreten.

Durch den Einsatz einer oder mehrerer beispielhaft in Figur 6 gezeigten Abstrahlvorrichtungen 44 ohne eigenen Reflektor bzw. ohne Parabolspiegel, kann in der Regel keine Verbesserung der Strahlenführung erreicht werden, die Herstellungskosten der Desinfektionsvorrichtung können hingegen reduziert werden.

Eine oder mehrere Abstrahlvorrichtungen 40, die gemäß dem in Figur 4 gezeigten Ausführungsbeispiel ausgebildet sind, können vorteilhaft in einer Desinfektionsvorrichtung 10 gemäß dem in Figur 1 gezeigten Ausführungsbeispiel eingesetzt werden. Vorzugsweise ist der Reflektor 62 der Abstrahlvorrichtung 40 dabei als Parabolspiegel mit zumindest einer im Brennpunkt angeordneten Strahlungsquelle 60 ausgebildet, die in den Parabolspiegel hineinstrahlt. Als Strahlungsquelle 60 wird vorzugsweise eine UVC-LED verwendet. Der Parabolspiegel kann dabei z.B. eine sphärische Konizität von -1 aufweisen. Der Aperturradius des Parabolspiegels kann dabei beispielsweise so gewählt werden, dass die Aperturfläche der Reflektoren 62 aller verwendeten Abstrahlvorrichtungen 40 vorzugsweise weniger als ca. 20%, und weiterhin vorzugsweise weniger als 3% der Aperturfläche der als Hohlspiegel ausgebildeten Reflexionsfläche 35 an der Innenseite des Bodenteils 26 des Druckgefäßes 12 beträgt. Der sphärische Radius ist mit Vorteil derart angepasst, dass verhältnismäßig wenig Strahlungsverlust an der zylindrischen Oberfläche des Druckgefäßes 12 auftritt.

Die Einbauposition der Abstrahlvorrichtungen 40 befindet sich vorzugsweise in der Nähe einer endseitigen Reflexionsfläche 35 des Druckgefäßes 12. Die Strahlung der Abstrahlvorrichtungen 40 ist vorzugsweise nahezu parallel zur Längsachse des Druckgefäßes 12 auf die gegenüberliegende Reflexionsfläche 34 gerichtet.

Da in der Gesamtanordnung ein Verlust der von der gegenüberliegende Reflexionsfläche 34 zurückreflektierten Strahlung beim Auftreffen auf dieselbe oder auf eine andere Abstrahlvorrichtung 40 durch eine leichte Abweichung von dieser Parallelität gemindert werden kann, und dabei gleichzeitig eine weniger konzentrierte, also besser verteilte Bestrahlung des Luftvolumens erfolgen kann, kann es von Vorteil sein, zumindest eine Abstrahlvorrichtung 40 außerhalb der Längsachse des Druckgefäßes 12 anzuordnen. Um eine bessere Bestrahlung des gesamten Innenraums 50 zu erreichen kann es weiterhin von Vorteil sein, entsprechend mehrere Abstrahlvorrichtungen 40 im Druckgefäß 12 vorzusehen.

Vorzugsweise kann zumindest eine Abstrahlvorrichtung 40 außerhalb der Längsachse des Druckgefäßes 12angeordnet sein. Weiterhin vorzugsweise kann an jeder in Form eines Hohlspiegels ausgebildeten Reflexionsfläche 34, 35 die gleiche ungerade Anzahl von sich nicht gegenüberliegenden Abstrahlvorrichtungen 40 vorgesehen sein. Weiterhin vorzugsweise können zwei Abstrahlvorrichtungen 40 unter einer Winkelposition von 90° +/- 45° bezüglich der Längsachse des Druckgefäßes 12 vorgesehen sein. Weiterhin vorzugsweise können z.B. drei Abstrahlvorrichtungen 40 an einer in Form eines Hohlspiegels ausgebildeten Reflexionsfläche 34, 35 vorgesehen sein. Die Abstrahlvorrichtungen 40 können mit Vorteil dezentral mit einem Abstand vom ca. 0,25-fachen bis zum ca. 0,75-fachen, vorzugsweise ungefähr dem 0,6-fachen des Radius der Apertur des die Reflexionsfläche 34, 35 ausbildenden Hohlspiegels außerhalb der Längsachse des Druckgefäßes 12 angeordnet sein.

Die Abstrahlvorrichtungen 40 strahlen grundsätzlich nahezu parallel zur Längsachse des Druckgefäßes 12, vorzugsweise jedoch leicht der Hohlspiegelkrümmung entsprechend in Richtung der Längsachse geneigt und weiterhin vorzugsweise zusätzlich mit einer zum dem Zylindermantel tangentialen Richtungskomponente, so dass eine bessere zirkulare Durchstrahlung des Innenraums 50 bewirkt wird und Strahlungsverluste des Sekundärstrahls durch Auftreffen auf Flächen der mindestens einen Abstrahlvorrichtung 40 vermindert werden.

Die mindestens eine Abstrahlvorrichtung 40 kann innerhalb des Hohlspiegels eines Bodenteils 26 angeordnet sein, wobei vorzugsweise der äußere Rand des Reflektors 62 der Abstrahlvorrichtung 40 auf der Oberfläche des Hohlspiegels angeordnet sein kann. Dabei kann der Hohlspiegel und das mit Hohlspiegel verbundene Bodenteil 26 in diesem Bereich einen Durchbruch für Revisions- und/oder Reparaturzwecke aufweisen.

Bei einer Desinfektionsvorrichtung 10 mit einem rohrförmigen Druckbehälter 16, 18, wie beispielsweise in Figur 2 und 3 dargestellt, kann der als Hohlspiegel ausgebildete Reflektor 62 (siehe Figuren 2 bis 5) der Abstrahlvorrichtung 42, 46 vorzugsweise derart dimensioniert sein, dass die Apertur des Reflektors 62 das ca. 0,5- bis ca. 2fache des Radius des Reflektors 62 beträgt, wobei die Konizität des Reflektors 62 ca. -1 betragen kann. Dieser kann also in beiden Fällen zumindest näherungsweise als Parabolspiegel ausgebildet sein.

Der Radius R des durch die Reflexionsfläche 36, 38 im Endteil 30, 32 des Druckgefäßes 12 ausgebildeten Hohlspiegels kann vorzugsweise dimensioniert werden als Radius R = L / x, wobei L der Länge des Druckgefäßes 12 entspricht und x beispielsweise in einem Bereich zwischen -0,1 und + 1,0, vorzugsweise zwischen ca. +0,2 und ca. 1,0 liegt.

Vorzugsweise kann eine Desinfektionsvorrichtung 10 mit einem rohrförmigen Druckbehälter 16, 18 mit ein bis fünf oder mehr Strahlungsquellen 60 ausgestattet sein. Dabei können die Strahlungsquellen 60 beispielsweise als LEDs bzw. LED-Chips ausgebildet sein. Die Strahlungsquellen 60 können beispielsweise zentral oder kreisförmig mit einem Radius r um die Längsachse des rohrförmigen Druckbehälters 16, 18 angeordnet sein. Der Radius r kann dabei vorzugsweise und ungefähr als r < 0,3 R in Abhängigkeit des Radius R des Hohlspiegels bestimmt werden. Der Abstand d der mindestens einen Strahlungsquelle 60 vom Hohlspiegel in Richtung der Längsachse des Druckbehälters 16, 18 kann beispielsweise als ca. 0,5 R in Abhängigkeit des Radius R des Hohlspiegels bestimmt werden.

Die in den Abstrahlvorrichtungen 40, 42, 44, 46 eingesetzten Strahlungsquellen 60 geben vorzugsweise UV-Strahlung ab. Besonders wirksam ist die erfindungsgemäße Desinfektion, wenn Strahlung mit Wellenlängen zwischen ca. 200 nm und ca. 300 nm abgegeben wird. Derartige Strahlung wirkt stark bakterizid. Sie wird von der DNA absorbiert, zerstört deren Struktur und inaktiviert lebende Zellen. Bindungen innerhalb der DNA-Stränge von Viren, Bakterien und Schimmelsporen werden durch die Strahlung zerstört. Mikroorganismen wie Viren, Bakterien, Hefen und Pilze, können mittels UV-Strahlung in kurzer Zeit unschädlichgemacht werden. Bei ausreichend hoher Bestrahlungsstärke ist die UV-Desinfektion besonders zuverlässig. Darüber hinaus können Mikroorganismen keine Resistenz gegen UV-Strahlen entwickeln.

Da bei der Bestrahlung keine Chemikalien zur Desinfektion der Luft verwendet werden, ist diese Art der Desinfektion umweltfreundlich. Im Übrigen ist sie auch für Personen unbedenklich, sofern anwesende Personen von der Strahlung abgeschirmt sind. Eine zuverlässige Abschirmung kann beispielsweise bereits durch das Gehäuse des Druckgefäßes 12 erfolgen.

Das Druckgefäß 12 weist Materialien auf, die gegenüber der von den Strahlungsquellen 60 abgegebenen Strahlung stabil sind.

Weiterhin vorzugsweise geben die Strahlungsquellen 60 eine UVC-Strahlung ab. UVC-Strahlung ist besonders gut für die Luftentkeimung geeignet. Wenn UVC-Strahlung auf Mikroorganismen trifft, so wird deren DNA innerhalb kürzester Zeit so verändert, dass eine Reproduktion nicht mehr möglich ist. Derart können beispielsweise Viren und Keime wie MRSA, SARS, Corona oder H7N9 besonders schnell und wirksam eliminiert werden. Dabei liegt der wirksame Wellenlängenbereich vorzugsweise zwischen ca. 250 nm und ca. 280nm.

So kann beispielsweise die in einem Drucklufttank befindliche Luft mit Hilfe von UVC-Strahlung entkeimt werden, indem in der Luft schwebende sowie sich an der Tankinnenwand befindliche Mikroorganismen durch die UVC-Strahlung erreicht und abgetötet werden. Die Strahlendosis, die zur Abtötung von Mikroorganismen und Viren nötig ist, liegt beispielsweise zwischen ca. 2,5 - 5,0 mW sec/cm² (für Staphylococcus aureus) und ca. 34 mW sec/cm² (für den Hepatitisvirus).

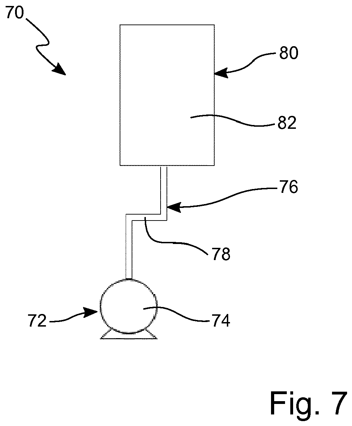

Figur 7 zeigt beispielhaft ein Druckluftsystem für medizinische oder dentalmedizinische Anwendungen. In schematischer Darstellung ist hier ein Kompressor 72 gezeigt, der über eine Verbindungsleitung 76, beispielsweise ein Rohr, mit einer Behandlungseinrichtung 80, beispielsweise ein Behandlungsstuhl, verbunden ist. Eine erfindungsgemäße Desinfektionsvorrichtung 10 kann dabei beispielsweise Luftentkeimung im Drucktank 74 des Kompressors 72 vorgesehen sein. Alternativ oder zusätzlich kann die Behandlungseinrichtung 80 mit einer oder mehreren Drucklufteinrichtungen 82 ausgestattet sein, die eine erfindungsgemäße Desinfektionsvorrichtung 10 aufweisen. Alternativ oder zusätzlich kann die Verbindungsleitung 76 ein oder mehrere Rohrabschnitte 78 mit einer erfindungsgemäßen Desinfektionsvorrichtung 10 aufweisen.

Desinfektionsvorrichtungen 10 können einfach oder mehrfach in einer Verbindungsleitung 76 und/oder in einer Behandlungseinrichtung 80 vorgesehen sein. Dabei kann eine Desinfektionsvorrichtung 10 bei entsprechender Dimensionierung auch als zusätzlicher Puffer für zu desinfizierende Luft dienen. Derart kann gewährleistet werden, dass auch vorübergehende, d.h. verhältnismäßig kurze, Spitzenflüsse des Mediums sicher desinfiziert werden. Eine geeignete Dimensionierung zum Bereitstellen eines derartigen Puffers kann bereits durch eine verhältnismäßig geringe Vergrößerung des Durchmessers eines rohrförmigen Druckbehälters 16, 18 erreicht werden.

Zur Leistungssteigerung können beispielsweise mehrere Desinfektionsvorrichtungen 10 in Serie oder parallel angeordnet werden.

Die anhand der Ausführungsbeispiele beschriebenen Desinfektionsvorrichtungen 10 sind besonders geeignet zur Desinfektion von Druckluft in Druckluftspeichern bzw. Druckluftgefäßen in der Medizin oder Dentalmedizin. Eine erfindungsgemäße Desinfektionsvorrichtung 10 kann auch zur Desinfektion anderer Medien wie z.B. Flüssigkeiten eingesetzt werden. Gegebenenfalls können hierzu entsprechende konstruktive Abwandlungen zweckmäßig sein. Voraussetzung für die Wirksamkeit der Desinfektionsvorrichtung 10 ist, dass die Reichweite der Strahlung im Medium unter Berücksichtigung der Abmessungen des Druckgefäßes 12 hinreichend groß ist.

Ein Druckluftspeicher für dentale Behandlungsluft kann beispielsweise abhängig von Parametern wie Luftverbrauch, Strahlungsintensität und Baugröße innerhalb oder außerhalb einer dentalen Behandlungseinrichtung 80, z.B. ein Behandlungsstuhl oder ein Stuhlmodul, angeordnet sein.

Als Strahlungsquellen 60 können besonders vorteilhaft eine oder mehrere UVC-LEDs vorgesehen sein, die vorzugsweise Strahlung mit einem Wellenlängenbereich von ca. 250 nm bis ca. 280 nm abgeben. Die als UVC-LEDs ausgebildeten Strahlungsquellen 60 sind jeweils an geeigneter Position in einem Druckluftspeichers angeordnet und geben ihre Strahlung in eine Reflexionseinrichtung ab, welche derart ausgestaltet ist, dass die Strahlung möglichst weite Streckenabschnitte bis zur nächsten Reflexion durchläuft. Durch möglichst lange frei von Strahlungsreflexion zurückgelegte Streckenabschnitte können die Strahlungsverluste reduziert werden, die bedingt durch eine Reflexion auftreten. Derart kann ein Wirkungsmultiplikator von ca. 3 bis 8 (oder noch höher falls bessere Reflektoren zur Verfügung stehen) in der Desinfektionsvorrichtung 10 erzielt werden, wodurch die Anzahl der zur Bestrahlung notwendigen UVC-LEDs erheblich reduziert werden kann.

Um eine zuverlässige Desinfektion zu gewährleisten, ist es notwendig, dass alle Luftschichten und Oberflächen, die sich innerhalb des Druckgefäßes 12 befinden vom Strahlungsbereich der UVC-Strahler erfasst werden. Die Verweilzeit des Mediums im Druckgefäß 12 und die Strahlungsintensität müssen derart aufeinander abgestimmt sein, dass die zur sicheren Abtötung etwaiger Mikroorganismen im Medium notwendige Strahlendosis möglichst durchgehend erreicht wird.

Bei der Verwendung von derzeit zur Verfügung stehenden UVC-Leuchtdioden steigen mit höherer Strahlungsleistung auch Kosten und Aufwand deutlich. Die Verwendung von in verschiedenen anderen Anwendungsgebieten an und für sich gebräuchlichen Quecksilberdampflampen als Strahlungsquelle ist in einem Druckbehälter für Druckluft, welche bei Zahnpatienten der Mundhöhle angewandt wird, nicht erstrebenswert.

Bei Desinfektionsvorrichtungen 10, wie sie in den Figuren 1 bis 3 beispielhaft gezeigt sind, kann zumindest weitestgehend vermieden werden, dass von einer Strahlungsquelle 60 abgegebene Strahlung an der Behälterwand des Druckgefäßes 12, die der Strahlungsquellen 60 gegenüber liegt, zu einem Großteil nutzlos absorbiert wird. Der höchste Nutzungsgrad bei der Desinfektion wird dann erzielt, wenn jeder von einer Strahlungsquelle 60 abgegebene Strahl die größtmögliche Entfernung zurücklegt, bzw. die größtmögliche Luftmenge ungehindert durchstrahlt, bevor er vollständig absorbiert wird. Die in einem Druckbehälter komprimierte Luft ist somit bereits ein Multiplikator für die Effizienz der Desinfektion. Unter Ausnutzung dieses durch die Erfinder erkannten Prinzips, kann auch die Strahlungsmenge einer verhältnismäßig kleinen UVC-LED in verhältnismäßig kurzer Zeit eine deutlich größere Menge an Keimen abtöten als ohne Berücksichtigung dieses Prinzips zu erwarten wäre.

Die Art und Anzahl der UVC-LEDs, die in geeigneter Weise in einem Druckgefäß 12 vorzusehen sind, kann unter Berücksichtigung von Parametern wie Tankgröße, Druck, Fördervolumen und Luftentnahme bestimmt werden. Dabei wirkt sich die Luftentnahme beispielsweise auf die durchschnittliche Verweilzeit der Luft in einem Drucklufttank aus.

Bei den vorangehend beschriebenen Desinfektionsvorrichtungen 10 wird eine möglichst vorteilhaft ausgebildete Innenverspiegelung eines Druckluftbehälters und eine möglichst vorteilhafte Anordnung der UVC-LEDs angestrebt, um eine vielfache Reflexion in Verbindung mit einer möglichst hohen dazwischenliegen Luftmasse für die von der/den UVC-LEDs ausgesendeten Strahlung zu erreichen. Dies ist insbesondere deshalb von hoher Bedeutung für die Wirksamkeit der Desinfektionsvorrichtung 10, da der Strahlungsverlust bei jeder Reflexion bei den gegenwärtig zur Verfügung stehenden Materialien bei bis zu fast ca. 10 % liegen kann.

Sendet man einen parallelen Lichtstrahl zwischen zwei Reflektoren beliebig oft hin und her, so entspricht der maximal erzielbare integrale Strahlungsgewinn dem Faktor des Kehrwertes des Reflexionsverlustes der Reflexionsfläche. Aufgrund der erfindungsgemä-ßen Vervielfachung der Strahlungswirkung wird eine wirtschaftliche und zuverlässige Umsetzung einer Luftdesinfektion in Druckbehältern unter Verwendung von UVC-LEDs ermöglicht. Es ist so beispielsweise möglich, mit beispielsweise einer nur einstelligen Anzahl von UVC-LEDs eine ausreichende Wirkung für eine Vielzahl von Anwendungen zu erzielen.

In alternativer Ausgestaltung können als ein oder mehrere Strahlungsquellen auch Laserdioden verwendet werden, vorzugsweise mit einer Emissionswellenlänge im Bereich zwischen ca. 250nm und ca. 280 nm. Vorteilhaft bei der Verwendung von Laserdioden ist, dass das Laserlicht noch zielgerichteter angewendet werden kann. Allerdings befinden sich Laserdioden in diesem Wellenlängenbereich derzeit noch in der Entwicklung und stehen nicht ohne weiteres für die industrielle Anwendung zur Verfügung.

Ein Gedanke, welcher der Erfindung zugrunde liegt, lässt sich wie folgt zusammenfassen: Die Erfindung betrifft eine Desinfektionsvorrichtung 10 mit einem Druckgefäß 12, das einen Innenraum 50 zur Aufnahme eines Mediums aufweist, und mit mindestens einer Abstrahlvorrichtung 40, 42, 44, 46 die derart angeordnet ist, dass sie Strahlung in den Innenraum 50 des Druckgefäßes 12 abgibt, wobei ein oder mehrere Oberflächen des Innenraums 50 des Druckgefäßes 12 zumindest teilweise als Reflexionsfläche 34, 35, 36, 37, 38, 39, 52 für die Strahlung ausgebildet sind. Erfindungsgemäß wird eine besonders zuverlässige Entkeimung von medizinischer oder dentalmedizinischer Druckluft im Druckgefäß 12 ermöglicht. Ein oder mehrere erfindungsgemäße Desinfektionsvorrichtungen 10 können in einem Kompressor 72 zum Bereitstellen von Druckluft für dentalmedizinische Anwendungen, in einer dentalen Behandlungseinrichtung 80 mit einer Drucklufteinrichtung 82 und/oder in einer Verbindungseinrichtung mit einem oder mehreren Rohrabschnitten 78 zum Führen von Druckluft für dentalmedizinische Anwendungen vorgesehen sein. Weitere Vorteile der erfindungsgemäßen Desinfektionsvorrichtungen 10 sind unter anderem: Reduktion von Geschmacks- und Geruchsbeeinträchtigung, Vermeidung der Bildung von gesundheitsgefährdenden Nebenprodukten, möglicher Verzicht auf die Verwendung von Quecksilber zur Strahlungserzeugung, möglicher Verzicht auf die Zugabe von Chemikalien zur Desinfektion, geringer Wartungsbedarf und einfache Handhabung, niedrige Betriebskosten, sehr hohe Betriebssicherheit.

## Patentansprüche

1. Desinfektionsvorrichtung mit mindestens einer Abstrahlvorrichtung,
wobei
die Desinfektionsvorrichtung (10) ein rohrförmiges Druckgefäß (12) mit einem Innenraum (50) zur Aufnahme eines Mediums aufweist, und
die mindestens eine Abstrahlvorrichtung (40, 42, 44, 46) derart angeordnet ist, dass sie Strahlung in den Innenraum (50) abgibt,
wobei eine oder mehrere Oberflächen des Innenraums (50) zumindest teilweise als Reflexionswobei
fläche (34, 35, 36, 37, 38, 39, 52) ausgebildet sind, die Reflexionsfläche (34, 35, 36, 37, 38, 39, 52) durch den Einbau einer Spiegelfolie und/oder eines Spiegelblechs oder durch Bedampfen hergestellt ist, wobei
die mindestens eine Abstrahlvorrichtung (40, 42, 44, 46) eine UVC-LED-Strahlungsquelle (60) aufweist, **dadurch gekennzeichnet, dass**
die Abstrahlvorrichtung (44) derart ausgebildet ist, dass eine Hauptrichtungskomponente der abgegebenen Strahlung parallel zu einer Längsachse des Druckgefäßes (12) ausgerichtet ist, wobei
die mindestens eine Abstrahlvorrichtung (40, 42, 44, 46) im Bereich einer ersten Seite des Innenraums (50) angeordnet ist, wobei
eine zweite, dieser ersten Seite entlang der Längsachse betrachtet gegenüberieqpnde Seite des Innenraums (50) eine Reflexionsfläche (34, 36, 38) für die Strahlung aufweist.

2. Desinfektionsvorrichtung nach Anspruch 1, **dadurch gekennzeichnet, dass** die Strahlungsquelle der Abstrahlvorrichtung (40, 42) in einem Hohlspiegel angeordnet ist.

3. Desinfektionsvorrichtung nach einem der Ansprüche 1 bis 2, **dadurch gekennzeichnet, dass** mindestens zwei sich gegenüberliegende Seiten des Innenraums (50) Reflexionsflächen (34, 35, 36, 37, 38, 39, 52) für die Strahlung aufweisen.

4. Desinfektionsvorrichtung nach Anspruch 1 oder 3, **dadurch gekennzeichnet, dass** mindestens eine der Reflexionsflächen (34, 35, 36, 37, 38, 39) in Form eines Hohlspiegels ausgebildet ist.

5. Desinfektionsvorrichtung nach einem der Ansprüche 1 bis 4 **dadurch gekennzeichnet, dass** mindestens eine Reflexionsfläche (34, 35, 36, 37, 38, 39, 52) durch eine auf ein Trägermaterial aufgebrachte Reflexionsschicht ausgebildet ist.

6. Desinfektionsvorrichtung nach einem der Ansprüche 1 bis 5 **dadurch gekennzeichnet, dass** das Druckgefäß (12) als Tank (14) mit mindestens einem Durchlass (20) zum Ein- und Ausströmen des Mediums ausgebildet ist.

7. Desinfektionsvorrichtung nach einem der Ansprüche 1 bis 6 **dadurch gekennzeichnet, dass** das Druckgefäß (12) als rohrförmiger Druckbehälter (16, 18) mit zwei oder mehr Durchlässen (20) für das Medium ausgebildet ist.

8. Desinfektionsvorrichtung nach einem der Ansprüche 1 bis 7, **dadurch gekennzeichnet, dass** das Druckgefäß (12) als Druckluftgefäß für dentale Druckluft ausgebildet ist.

9. Kompressorsystem zum Bereitstellen von Druckluft für dentalmedizinische Anwendungen, **dadurch gekennzeichnet, dass** das Kompressorsystem (72) eine Desinfektionsvorrichtung (10) nach einem der Ansprüche 1 bis 8 aufweist.

10. Dentale Behandlungseinrichtung mit einer Drucklufteinrichtung, **dadurch gekennzeichnet, dass** die Drucklufteinrichtung (82) eine Desinfektionsvorrichtung (10) nach einem der Ansprüche 1 bis 8 aufweist.

11. Verbindungseinrichtung (76) mit einem oder mehreren Rohrabschnitten zum Führen von Druckluft für dentalmedizinische Anwendungen, **dadurch gekennzeichnet, dass** mindestens ein Rohrabschnitt (78) eine Desinfektionsvorrichtung (10) nach einem der Ansprüche 1 bis 8 aufweist.

## Claims

1. Disinfection device with at least one radiating device,
wherein
the disinfection device (10) has a tubular pressure vessel (12) with an interior space (50) for receiving a medium, and
the at least one radiating device (40, 42, 44, 46) is arranged such that it emits radiation into the interior space (50),
wherein
one or several surfaces of the interior space (50) are formed at least partially as a reflection surface (34, 35, 36, 37, 38, 39, 52), wherein the reflection surface (34, 35, 36, 37, 38, 39, 52) is produced by installing a mirror foil and/or a mirror sheet or by vapor deposition, wherein
the at least one radiating device (40, 42, 44, 46) comprises a UVC LED radiation source (60), **characterised in that**
the radiating device (44) is designed such that a principal directional component of the emitted radiation is aligned parallel to a longitudinal axis of the pressure vessel (12), wherein
the at least one radiating device (40, 42, 44, 46) is arranged in the region of a first side of the interior space (50), wherein
a second side of the interior space (50), opposite this first side as viewed along the longitudinal axis, comprises a reflection surface (34, 36, 38) for the radiation.

2. Disinfection device according to claim 1, **characterised in that** the radiation source of the radiating device (40, 42) is arranged in a concave mirror.

3. Disinfection device according to one of claims 1 to 2, **characterised in that** at least two sides of the interior space (50) opposite to one another comprise reflection surfaces (34, 35, 36, 37, 38, 39, 52) for the radiation.

4. Disinfection device according to claim 1 or 3, **characterised in that** at least one of the reflection surfaces (34, 35, 36, 37, 38, 39) is formed in the form of a concave mirror.

5. Disinfection device according to one of claims 1 to 4, **characterised in that** at least one reflection surface (34, 35, 36, 37, 38, 39, 52) is formed by a reflection layer applied to a substrate.

6. Disinfection device according to one of claims 1 to 5, **characterised in that** the pressure vessel (12) is designed as a tank (14) with at least one passage (20) for inflow and outflow of the medium.

7. Disinfection device according to one of claims 1 to 6, **characterised in that** the pressure vessel (12) is designed as a tubular pressure container (16, 18) with two or more passages (20) for the medium.

8. Disinfection device according to one of claims 1 to 7, **characterised in that** the pressure vessel (12) is configured as a compressed air vessel for dental compressed air.

9. Compressor system for providing compressed air for dental applications, **characterised in that** the compressor system (72) comprises a disinfection device (10) according to one of claims 1 to 8.

10. Dental treatment device with a compressed air device, **characterised in that** the compressed air device (82) comprises a disinfection device (10) according to one of claims 1 to 8.

11. Connection device (76) with one or several tube sections for guiding compressed air for dental medical applications, **characterised in that** at least one tube section (78) comprises a disinfection device (10) according to one of claims 1 to 8.

## Revendications

1. Dispositif de désinfection comportant au moins un dispositif de rayonnement, dans lequel
le dispositif de désinfection (10) comprend un récipient sous pression (12) de forme tubulaire avec un espace intérieur (50) destiné à recevoir un milieu, et
ledit au moins un dispositif de rayonnement (40, 42, 44, 46) est disposé de manière à émettre un rayonnement dans l'espace intérieur (50),
dans lequel une ou plusieurs surfaces de l'espace intérieur (50) sont réalisées au moins partiellement sous forme de surface de réflexion (34, 35, 36, 37, 38, 39, 52), la surface de réflexion (34, 35, 36, 37, 38, 39, 52) étant réalisée par l'installation d'un film miroir et/ou d'une tôle miroir ou par vaporisation,
dans lequel ledit au moins un dispositif de rayonnement (40, 42, 44, 46) comprend une source de rayonnement LED UVC (60),
**caractérisé en ce que** le dispositif de rayonnement (44) est réalisé de telle sorte qu'une composante directionnelle principale du rayonnement émis est orientée parallèlement à un axe longitudinal du récipient sous pression (12),
dans lequel ledit au moins un dispositif de rayonnement (40, 42, 44, 46) est disposé dans la zone d'un premier côté de l'espace intérieur (50),
dans lequel un deuxième côté de l'espace intérieur (50), opposé à ce premier côté vu le long de l'axe longitudinal, comprend une surface de réflexion (34, 36, 38) pour le rayonnement.

2. Dispositif de désinfection selon la revendication 1, **caractérisé en ce que** la source de rayonnement du dispositif de rayonnement (40, 42) est disposée dans un miroir concave.

3. Dispositif de désinfection selon l'une quelconque des revendications 1 à 2, **caractérisé en ce qu'**au moins deux côtés opposés de l'espace intérieur (50) comprennent des surfaces de réflexion (34, 35, 36, 37, 38, 39, 52) pour le rayonnement.

4. Dispositif de désinfection selon la revendication 1 ou 3, **caractérisé en ce qu'**au moins une des surfaces de réflexion (34, 35, 36, 37, 38, 39) est réalisée sous la forme d'un miroir concave.

5. Dispositif de désinfection selon l'une quelconque des revendications 1 à 4, **caractérisé en ce qu'**au moins une surface de réflexion (34, 35, 36, 37, 38, 39, 52) est formée par une couche de réflexion appliquée sur un matériau support.

6. Dispositif de désinfection selon l'une quelconque des revendications 1 à 5, **caractérisé en ce que** le récipient sous pression (12) est réalisé sous la forme d'un réservoir (14) avec au moins un passage (20) pour l'entrée et la sortie du milieu.

7. Dispositif de désinfection selon l'une quelconque des revendications 1 à 6, **caractérisé en ce que** le récipient sous pression (12) est réalisé sous la forme d'un récipient sous pression tubulaire (16, 18) comportant deux ou plusieurs passages (20) pour le milieu.

8. Dispositif de désinfection selon l'une quelconque des revendications 1 à 7, **caractérisé en ce que** le récipient sous pression (12) est réalisé sous la forme d'un récipient d'air comprimé pour air comprimé dentaire.

9. Système de compresseur pour la mise à disposition d'air comprimé pour des applications dentaires, **caractérisé en ce que** le système de compresseur (72) comprend un dispositif de désinfection (10) selon l'une quelconque des revendications 1 à 8.

10. Équipement de traitement dentaire comportant un système d'air comprimé, **caractérisé en ce que** le système d'air comprimé (82) comprend un dispositif de désinfection (10) selon l'une quelconque des revendications 1 à 8.

11. Équipement de raccordement (76) comportant une ou plusieurs sections de tuyau pour amener de l'air comprimé pour des applications dentaires, **caractérisé en ce qu'**au moins une section de tuyau (78) comprend un dispositif de désinfection (10) selon l'une quelconque des revendications 1 à 8.
